Europäisches Patentamt

European Patent Office

Office européen des brevets

(11) Publication number: **0 321 617 B1**

(12) # EUROPEAN PATENT SPECIFICATION

(45) Date of publication of patent specification: **07.10.92**  (51) Int. Cl.⁵: **A42B 1/24**, A42B 1/06,
//G02C3/02,A61F9/04

(21) Application number: **87311327.8**

(22) Date of filing: **22.12.87**

(54) **Cap with visor and pivotable eye shield.**

<table>
<tr><td>

(43) Date of publication of application:
**28.06.89 Bulletin 89/26**

(45) Publication of the grant of the patent:
**07.10.92 Bulletin 92/41**

(84) Designated Contracting States:
**AT BE CH DE ES FR GB IT LI LU NL SE**

(56) References cited:
**DE-B- 1 054 721**     **FR-A- 1 094 289**
**FR-A- 2 221 117**     **US-A- 1 485 842**
**US-A- 2 619 641**     **US-A- 2 654 089**
**US-A- 2 690 586**     **US-A- 3 273 163**

</td><td>

(73) Proprietor: **Day, Sheng-Tong**
**1 Chung Yang North Road**
**Ching Shui Taichung Hsien(TW)**

(72) Inventor: **Day, Sheng-Tong**
**1 Chung Yang North Road**
**Ching Shui Taichung Hsien(TW)**

(74) Representative: **Gordon, Michael Vincent et al**
**GILL JENNINGS & EVERY 53-64 Chancery Lane**
**London WC2A 1HN(GB)**

</td></tr>
</table>

Rank Xerox (UK) Business Services

## Description

The present invention relates to a cap provided with a visor, such a cap being known as a visor cap, and more particularly to a visor cap provided with a pivotable eye shield.

It is perhaps well known that many people who perform outdoor activities always use an eye shield such as sun glasses or goggles for protection to avoid dust from getting into the eyes and also wear a cap or hat with a visor for shading the eyes.

In the past, a number of devices have been proposed for attaching eye glasses to the visor of a cap, such as is disclosed in US-A-4541125 in which a clip part attaches the main eye glasses part to a cap visor whereby the eye glasses part is pivotally movable between operative and inoperative positions, with other devices having been disclosed in US-A-4304005 and US-A-4636048.

An object of the present invention is to provide a visor cap having a pivotable eye shield which cannot only be angularly adjusted but can be moved back and forth to adjust the distance thereof from the eyes.

It is also known from US-A-2654089, acknowledged in the pre-characterising portion of the following independent claim, for a visor cap to comprise a cap body, a forwardly projecting visor which is secured to the cap body, a mounting block which is reciprocally secured to the visor, and an eye shield which is pivotally secured to the mounting block, the attachment between the visor and the mounting block including a longitudinal groove, and the attachment between the eye shield and the mounting block including a detachable member.

The present invention is characterised in that said longitudinal groove is a dovetail groove integrally formed in a lower portion of the visor, and said detachable member is a transversely extending mounting shaft formed at an upper portion of the eye shield.

A visor cap in accordance with the present invention is not only useful but is considered to be stylish.

Preferably, the dovetail groove is open at its rear and an upper portion of the mounting block is formed of complementary trapezoidal shape for sliding frictional engagement within the dovetail groove.

Preferably, a lower portion of the mounting block is formed with a transversely extending retaining channel accessible through a narrower opening within which the mounting shaft is a snap fit.

More particularly, the eye shield includes a pair of upstanding legs whose upper ends are connected by the mounting shaft, the mounting block including a pair of slots extending in planes perpendicular to the mounting shaft for receiving respective ones of said legs.

The visor may be formed of a non-opaque plastics material whose rear edge is secured to the cap body, the mounting block may be formed of a resiliently deformable plastics material and the eye shield may be formed of a non-opaque plastics material in at least a lens portion thereof.

An example of a visor cap in accordance with the present invention will now be described in more detail with reference to the accompanying drawings in which:-

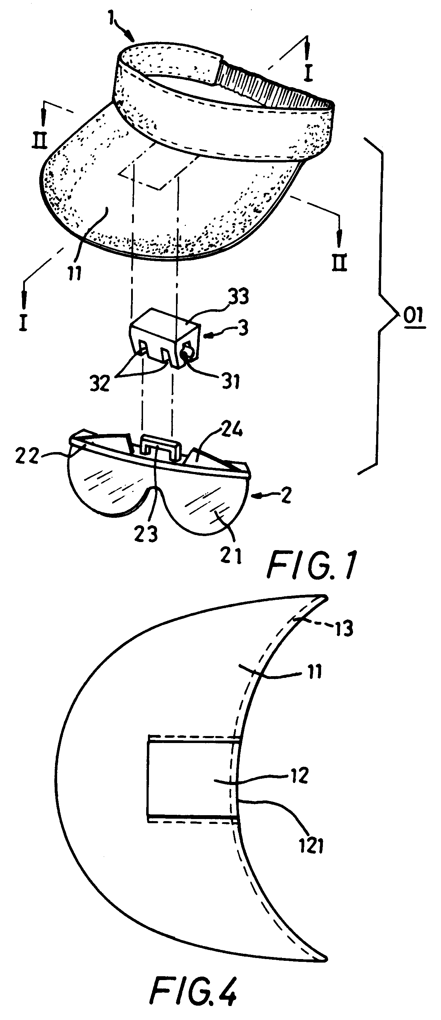

Figure 1 is a perspective, exploded view of a visor cap constructed in accordance with the present invention;

Figure 2 is a longitudinal sectional view of the visor cap in the assembled state taken along the line I-I of Figure 1;

Figure 3 is a transverse sectional view of the visor cap in the assembled state taken along the line II-II of Figure 1; and

Figure 4 is a bottom view of just the visor of the visor cap.

The visor cap of the present invention, designated generally by the numeral 01, comprises a cap body 1 having at least a visor 11, an eye shield 2 and a mounting block 3.

The cap body 1 may be of any type having the visor 11 attached thereto. The visor 11 may be integrally formed of a non-opaque plastics material and is to project forwardly of the cap body 1. Formed on the lower side middle portion of the visor 11 is a dovetail groove 12 which is directed longitudinally forwards from the rear edge of the visor 11 and defines an entry port 121 at its rear. The attachment groove 12 may alternatively be formed in a separate member affixed to the surface of the visor 11.

The eye shield 2, such as sun glasses or windshield goggles, has a single piece wind-shield lens 21 and a mounting frame 22 having in its lower end a groove 25 for frictional attachment thereto of the upper end of the lens 21. The mounting frame 22, on its upper side middle portion, is formed integrally with a transversely extending mounting shaft 23. More particularly, the eye shield 2 includes a pair of upstanding legs whose upper ends are connected by the mounting shaft 23, so that the legs and the mounting shaft when considered together are of inverted channel shape. On either side of the mounting shaft 23 is formed in a symmetrical manner one of a pair of triangular supplementary plates 24 to conform upwardly to the curve of the underside surface of the visor 11. It should be noted that the transverse mounting shaft 23 may alternatively be formed directly between to connect the pair of supplementary plates 24. Furthermore, the aforesaid mounting frame 22 having the mounting shaft 23 may alternatively be integrally formed with the lens 21 from a transparent plastics material.

The mounting block 3 is integrally moulded from a synthetic resin material, such as a resiliently deformable plastics material, of a trapezoidal shape provided at the shorter side end with an opening 31a. The opening 31a leads to a wider retaining channel 31 and a further narrow channel 31b. The opening 31a is substantially narrower in width than the mounting shaft 23 which can be snap inserted through the opening 31a into the retaining channel 31 to be pivotally movable therein. The mounting block 3 also includes a pair of slots 32 extending in planes perpendicular to the mounting shaft 23 for receiving respective ones of said legs at the ends of the mounting shaft 23. The mounting block 3 may omit the slots 32 if there are no legs or if the legs are to be disposed beyond the ends of the mounting block 3. The broader side end of the mounting block 3 is so configured that its base 33 can be engaged frictionally and moved back and forth in the above said groove 12.

In use and operation of the visor cap 01, when it is desired that the eye shield 2 be mounted on the cap body 1, the mounting block 3 is first attached by its base 33 to the groove 12 on the underside surface of the visor 11 through the entry port 121 located at the rear side of the groove 12. Following this arrangement, the mounting block 3 is now capable of being slid back and forth and of being frictionally retained at any selected position along the groove 12. The eye shield 2 is next mounted by snapping in position the mounting shaft 23 thereof in the retaining channel 31 of the mounting block 3, whereby the mounting shaft 23 is capable of being frictionally retained at any selected angle within the retaining channel 31 and is prevented by the relatively narrower mouthed opening 31a from easily falling out.

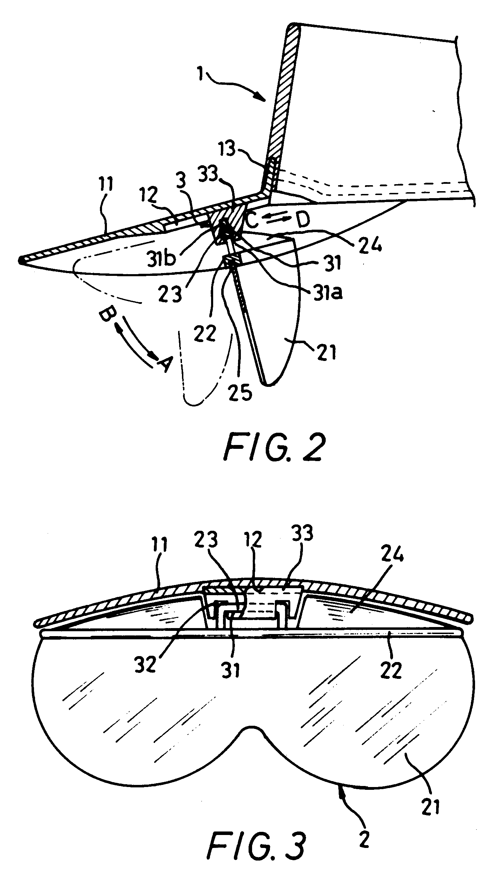

In this arrangement, depending on circumstances a user may desire to turn the lens 21 of the eye shield 2 downward in a direction as indicated by the arrow A in Figure 2 to be in a substantially vertical position relative to the visor 11 for use. While in this downward position of the lens 21, the eye shield 2 together with the mounting block 3 disposed thereabove may also be moved back and forth in directions as indicated by arrows C and D in Figure 2 to enable the wearer to appropriately adjust the distance between the lens 21 and the face. Angular adjustment of the lens 21 is possible, and to facilitate frictional retention the outer circumference of the mounting shaft 23 and the inner wall of the retaining channel 31 may be formed as circumferentially arranged and transversely extending series of interengagable teeth. Again, when not in use, the lens 21 may be raised directly upwardly in direction as indicated by the arrow B in Figure 2, thus permitting the lens 21 to abut against the underside of the visor 11.

The lens 21 may be separately disconnected and removed from the mounting block 3, or the lens 21 may alternatively be detached out together with the mounting block 3 from the visor 11, so that the cap body 1 can be separately used.

From the foregoing, it is seen that the preferred embodiment of the visor cap in accordance with the present invention provides an eye shield which can be readily attached to the bottom surface of the visor through the intermediary of the mounting block. When mounted in position on the visor, the eye shield lens can either be conveniently and quickly removed or when it is not in use the lens can be raised up to rest closely adjacent the underside of the visor. The design and construction of the mounting block permits ready attachment and detachment of the eye shield to and from the visor, as well as ready attachment and detachment of the two part relative to each other. The frictional engagement of the mounting block with the visor further allows the adjustment of distance to be made between the position of the eye shield and the eyes.

## Claims

1. A visor cap (01) comprising a cap body (1), a forwardly projecting visor (11) which is secured to the cap body (1), a mounting block (3) which is reciprocally secured to the visor (11), and an eye shield (2) which is pivotally secured to the mounting block (3), the attachment between the visor (11) and the mounting block (3) including a longitudinal groove (12), and the attachment between the eye shield (2) and the mounting block (3) including a detachable member (23), characterised in that said longitudinal groove is a dovetail groove (12) integrally formed in a lower portion of the visor (11), and said detachable member is a transversely extending mounting shaft (23) formed at an upper portion of the eye shield (2).

2. A visor cap according to claim 1, characterised in that the dovetail groove (12) is open at its rear (21) and an upper portion of the mounting block (3) is formed of complementary trapezoidal shape (33) for sliding frictional engagement within the dovetail groove (12).

3. A visor cap according to claim 1 or claim 2, characterised in that a lower portion of the mounting block (3) is formed with transversely extending retaining channel (31) accessible through a narrower opening (31a) within which the mounting shaft (23) is a snap fit.

4. A visor cap according to claim 3, characterised in that the eye shield (2) includes a pair of upstanding legs whose upper ends are connected by the mounting shaft (23), the mounting block (3) including a pair of slots (32) extending in planes perpendicular to the mounting shaft (23) for receiving respective ones of said legs.

5. A visor cap according to any preceding claim, characterised in that the visor (11) is formed of a non-opaque plastics material whose rear edge is secured to the cap body (1).

6. A visor cap according to any preceding claim, characterised in that the mounting block (3) is formed of a resiliently deformable plastics material.

7. A visor cap according to any preceding claim, characterised in that the eye shield (2) is formed of a non-opaque plastics material in at least a lens portion (21) thereof.

**Patentansprüche**

1. Schirmkappe (01) mit einem Kappenkörper (1), einem nach vorne vorstehenden Schirm (11), der am Kappenkörper (1) befestigt ist, einem Montageblock (3), der hin- und herbeweglich am Schirm (1) befestigt ist, und einem Augenschutz (2), der schwenkbar am Montageblock (3) befestigt ist, wobei die Befestigung zwischen dem Schirm (11) und dem Montageblock (3) eine Längsnut (12) und die Befestigung zwischen dem Augenschutz (2) und dem Montageblock (3) ein lösbares Element (23) umfaßt, dadurch gekennzeichnet, daß die Längsnut eine Schwalbenschwanznut (12) ist, die in einen unteren Abschnitt des Schirmes (11) integriert ist, und daß das lösbare Element ein quer verlaufender Montageschaft (23) ist, der an einem oberen Abschnitt des Augenschutzes (2) ausgebildet ist.

2. Schirmkappe nach Anspruch 1, dadurch gekennzeichnet, daß die Schwalbenschwanznut (12) an ihrer Rückseite (21) offen ist und daß ein oberer Abschnitt des Montageblocks (3) eine komplementäre trapezförmige Gestalt (33) für einen Gleitreibungseingriff in die Schwalbenschwanznut (12) besitzt.

3. Schirmkappe nach Anspruch 1 oder 2, dadurch gekennzeichnet, daß ein unterer Abschnitt des Montageblocks (3) mit einem quer verlaufenden Haltekanal (31) versehen ist, der durch eine engere Öffnung (31a) zugänglich ist, in der der Montageschaft (23) mittels Preßpassung befestigt ist.

4. Schirmkappe nach Anspruch 3, dadurch gekennzeichnet, daß der Augenschutz (2) ein Paar von aufrecht stehenden Schenkeln aufweist, deren obere Enden über den Montageschaft (23) miteinander verbunden sind, und daß der Montageblock (3) ein Paar von Schlitzen (32) aufweist, die sich in Ebenen senkrecht zum Montageschaft (23) zur Aufnahme von entsprechenden Schenkeln erstrecken.

5. Schirmkappe nach einem der vorangehenden Ansprüche, dadurch gekennzeichnet, daß der Schirm (11) aus einem nicht-opaken Kunststoffmaterial hergestellt ist, dessen hinterer Rand am Kappenkörper (1) befestigt ist.

6. Schirmkappe nach einem der vorangehenden Ansprüche, dadurch gekennzeichnet, daß der Montageblock (3) aus einem elastisch verformbaren Kunststoffmaterial besteht.

7. Schirmkappe nach einem der vorangehenden Ansprüche, dadurch gekennzeichnet, daß der Augenschutz (2) in mindestens einem optischen Abschnitt (21) desselben aus einem nicht-opaken Kunststoffmaterial besteht.

**Revendications**

1. Coiffure à visière comprend un corps de coiffure (1), une visière (11) se projetant vers l'avant qui est fixée au corps (1) de la coiffure, un bloc de montage (3) qui est fixé mobile en translation sur la visière, et une protection oculaire (2) qui est fixée de façon rabattable au bloc de montage (3), l'assemblage entre la visière (11) et le bloc de montage (3) comprenant une rainure longitudinale (12) et l'assemblage entre la protection oculaire (2) et le bloc de montage (3) comprenant un élément séparable (23), caractérisée en ce que ladite rainure longitudinale est une rainure à queue d'aronde (12) formée dans la matière d'une partie inférieure de la visière (11), et ledit élément séparable est une barrette de montage (23) orientée transversalement qui est formée au droit d'une partie supérieure de la protection oculaire (2).

2. Coiffure à visière selon la revendication 1, caractérisée en ce que la rainure à queue d'aronde (12) est ouverte à sa partie arrière (21) et une partie supérieure du bloc de montage (3) est d'une forme trapézoïdale complémentaire (33) pour s'engager dans la rainure à queue d'aronde (12) pour y coulisser avec frottement.

3. Coiffure à visière selon la revendication 1 ou la revendication 2, caractérisée en ce qu'une partie inférieure du bloc de montage (3) est munie d'une gorge de retenue (31) orientée transversalement, accessible à travers une ouverture (31a) dans laquelle la barrette de montage (23) se monte par encliquetage.

4. Coiffure à visière selon la revendication 3, caractérisée en ce que la protection oculaire (2) comprend deux branches dirigées vers le haut dont les extrémités supérieures sont reliées par la barrette de montage (23), le bloc de montage (3) présentant deux fentes (32) qui s'étendent dans des plans perpendiculaires à la barrette de montage (23) pour recevoir respectivement lesdites branches.

5. Coiffure à visière selon une quelconque des revendications précédentes, caractérisée en ce que la visière (11) est formée d'une matière plastique non opaque dont le bord arrière est fixé au corps (1) de la coiffure.

6. Coiffure à visière selon une quelconque des revendications précédentes, caractérisée en ce que le bloc de montage (3) est formé d'une matière plastique élastiquement déformable.

7. Coiffure à visière selon une quelconque des revendications précédentes, caractérisée en ce que la protection oculaire (2) est formée d'une matière plastique non opaque du moins dans sa partie (21) formant oculaire.

*FIG.1*

*FIG.4*

FIG. 2

FIG. 3